# EUROPÄISCHE PATENTSCHRIFT

(11) **EP 0 400 011 B1**
(45) Veröffentlichungstag und Bekanntmachung des Hinweises auf die Patenterteilung: **26.01.1994**
(21) Anmeldenummer: 89900024.4
(22) Anmeldetag: 29.11.1988
(51) Int. Cl.: C07C 215/30, C07C 229/38, C07C 215/54, C07C 217/62, C07C 255/25, C07C 215/68, C07C 225/16

(54) **NEUE PHENYLETHANOLAMINE**
NEW PHENYLETHANOLAMINES
PHENYLETHANOLAMINES

(43) Veröffentlichungstag der Anmeldung: 05.12.1990
(73) Patentinhaber: BOEHRINGER INGELHEIM VETMEDICA GMBH, 55216 Ingelheim (DE)
(72) Erfinder: HURNAUS, Rudolf, D-7950 Biberach 1 (DE); REIFFEN, Manfred, D-7950 Biberach 1 (DE); SAUTER, Robert, D-7958 Laupheim (DE); GRELL, Wolfgang, D-7950 Biberach 1 (DE); RUPPRECHT, Eckhard, D-7960 Aulendorf-Tannhausen (DE)
(86) Internationale Anmeldenummer: EP8801083
(87) Internationale Veröffentlichungsnummer: WO9006299

(56) Entgegenhaltungen:
- EP-A- 0 026 298
- EP-A- 0 070 134
- EP-A- 0 101 069
- EP-A- 0 233 686
- DE-A- 3 718 638

## Beschreibung

In der EP-A-0,070,134 werden u.a. N-Phenylalkyl-aminoethanole, die durch einen Benzyloxyrest substituiert sind, welche u.a. eine fettreduzierende Wirkung aufweisen, und in der EP-A-0,026,298 N-Phenyl-, N-Benzyl- und N-α,α-Dimethylphenylethyl-aminoethanole, deren Phenylkern unsubstituiert ist und welche eine leistungsfördernde und fettreduzierende Wirkung aufweisen, beschrieben.

Es wurde nun gefunden, daß die neuen N-[2-(3-Chlor-phenyl)-2-hydroxy-ethyl]-N-(1-methyl-2-phenyl-ethyl)-amine der allgemeinen Formel 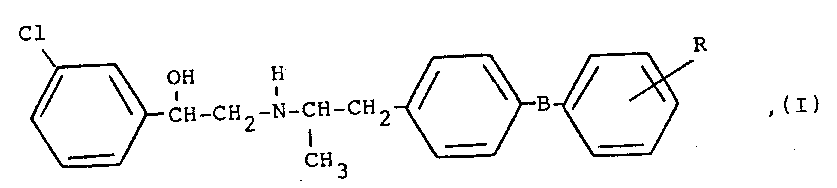
sich von den literaturbekannten Verbindungen durch den 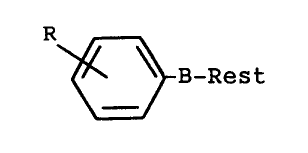
unterscheiden, wobei
B eine Bindung oder eine Methylengruppe und
R eine Methoxycarbonyl-, Ethoxycarbonyl-, 2-Hydroxy-ethoxy-, Methoxycarbonylmethoxy- oder Ethoxycarbonylmethoxygruppe in 2- oder 4-Stellung oder auch, wenn B eine Bindung darstellt und R in 4-Stellung steht, eine Carboxy- oder Carboxymethoxygruppe bedeuten, deren optische Isomere und deren Diastereomere sowie deren Additionssalze, insbesondere für die pharmazeutische Verwendung deren physiologisch verträgliche Additionssalze, wertvolle pharmakologische Eigenschaften aufweisen, nämlich eine Wirkung auf den Stoffwechsel, insbesondere eine blutzuckersenkende, körperfettreduzierende und Energieverbrauch-steigernde Wirkung, sowie eine Senkung der atherogenen Lipoproteine VLDL und LDL.

Außerdem können die neuen Verbindungen als Leistungsförderer bei Tieren, insbesondere zur Erzielung höherer täglicher Gewichtszunahmen und verbesserter Futterausnutzung in der Tierernährung, vorzugsweise in der Tiermast, eingesetzt werden, was ein weiterer Gegenstand der vorliegenden Erfindung ist.

Bevorzugte Verbindungen der Formel I sind jedoch folgende:
(a) 4'-[2-[N(2-(3-Chlor-phenyl)-2-hydroxy-ethyl)amino]propyl]-4-(2-hydroxy-ethoxy)biphenyl,
(b) 4'-[2-[N-(2-(3-Chlor-phenyl)-2-hydroxy-ethyl)amino]propyl]-biphenylyl-4-oxyessigsäure-methylester und
(c) 4'-[2-[N-(2-(3-Chlor-phenyl)-2-hydroxy-ethyl)amino]propyl]-diphenylmethan-2-carbonsäure-ethylester
sowie deren optische Isomere, deren Diastereomere und deren Säureadditionssalze.

Erfindungsgemäß erhält man die neuen Verbindungen nach folgenden Verfahren:
a) Umsetzung einer Verbindung der allgemeinen Formel 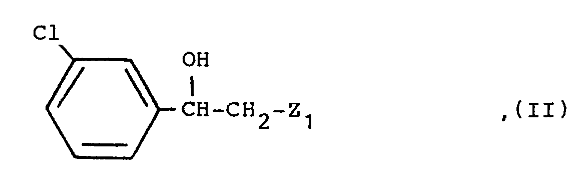 mit einer Verbindung der allgemeinen Formel 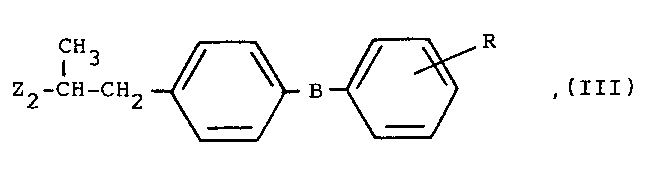 in denen
   R und B wie eingangs definiert sind,
   einer der Reste Z₁ oder Z₂ eine nukleophile Austrittsgruppe und
   der andere der Reste Z₁ oder Z₂ eine Aminogruppe bedeuten.
   Als nukleophile Austrittsgruppen kommen beispielsweise Halogenatome oder Sulfonyloxygruppen, z.B. ein Chlor-, Brom-oder Jodatom, die Methansulfonyloxy-, p-Toluolsulfonyloxy-oder Ethoxysulfonyloxygruppe, in Betracht.
   Die Umsetzung wird zweckmäßigerweise in einem Lösungsmittel oder Lösungsmittelgemisch wie Aceton, Diethylether, Acetonitril, Dimethylformamid, Dimethylsulfoxid, Methylenchlorid, Chloroform, Tetrahydrofuran, Dioxan oder in einem Überschuß der eingesetzten Verbindungen der allgemeinen Formeln II und/oder III und gegebenenfalls in Gegenwart eines säurebindenden Mittels, z.B. eines Alkoholats wie Kalium-tert.butylat, eines Alkalihydroxids wie Natrium- oder Kaliumhydroxid, eines Alkalicarbonats wie Kaliumcarbonat, einer tertiären organischen Base wie Triethylamin, N,N-Diisopropylethylamin oder Pyridin, wobei die letzteren gleichzeitig auch als Lösungsmittel dienen können, oder eines Reaktionsbeschleunigers wie Kaliumjodid je nach der Reaktionsfähigkeit des nukleophil austauschbaren Restes, zweckmäßigerweise bei Temperaturen zwischen 0 und 150°C, vorzugsweise bei Temperaturen zwischen 20 und 100°C, z.B. bei der Siedetemperatur des verwendeten Lösungsmittels, durchgeführt. Die Umsetzung kann jedoch auch ohne Lösungsmittel durchgeführt werden. Besonders vorteilhaft wird die Umsetzung jedoch in Gegenwart einer tertiären organischen Base oder eines Überschusses des eingesetzten Amins der allgemeinen Formel II oder III durchgeführt.
b) Reduktion einer gegebenenfalls im Reaktionsgemisch gebildeten Schiff'schen Base der allgemeinen Formel 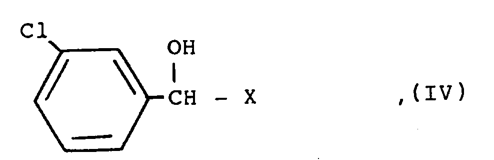 in der
   X eine Gruppe der Formel 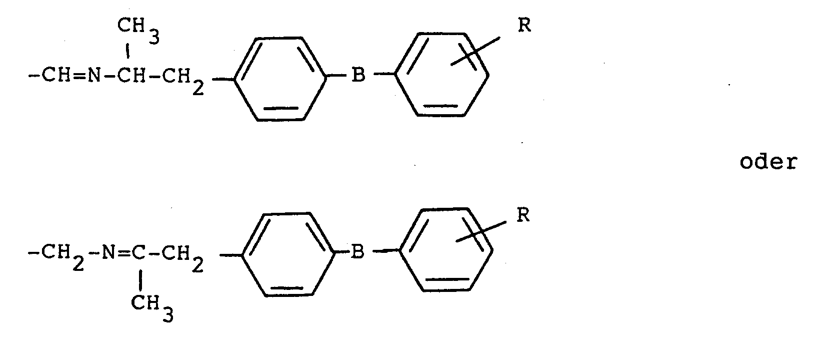 darstellt, wobei
   R und B wie eingangs definiert sind.
   Die Reduktion wird in einem geeigneten Lösungsmittel wie Methanol, Ethanol, Diethylether, Tetrahydrofuran, Dioxan, Essigsäure-ethylester oder Ethanol/Essigsäure-ethylester mit Wasserstoff in Gegenwart eines Hydrierungskatalysators wie Platin, Palladium/Kohle oder Raney-Nickel bei einem Wasserstoffdruck von 1 bis 5 bar oder mit einem Metallhydrid wie Lithiumaluminiumhydrid, Diboran, Natriumcyanborhydrid oder Boran/Dimethylsulfid, vorzugsweise jedoch mit Natriumborhydrid oder Natriumcyanborhydrid bei Temperaturen zwischen 0 und 50°C, vorzugsweise bei Raumtemperatur, durchgeführt. - Bei der Reduktion mit einem komplexen Metallhydrid wie Lithiumaluminiumhydrid, Diboran oder Boran/Dimethylsulfid, kann eine vorhandene Carbonylfunktion mitreduziert werden.
c) Reduktion einer gegebenenfalls im Reaktionsgemisch gebildeten Verbindung der allgemeinen Formel 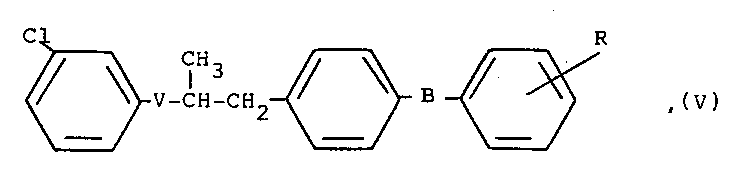 in der
   R und B wie eingangs definiert sind und
   V eine Gruppe der Formel 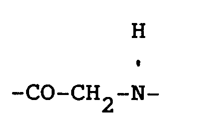 oder -CO-CH=N- darstellt.
   Die Reduktion wird vorzugsweise in einem geeigneten Lösungsmittel wie Methanol, Ethanol, Diethylether oder Tetrahydrofuran in Gegenwart eines Metallhydrids wie Natriumborhydrid, Lithiumaluminiumhydrid, Diboran, Boran/Dimethylsulfid oder Natriumcyanborhydrid, vorzugsweise jedoch mit Natriumborhydrid in Methanol oder Ethanol, zwischen 0 und 40°C, vorzugsweise jedoch bei Raumtemperatur, durchgeführt.
   Bei der Reduktion mit einem komplexen Metallhydrid wie Natriumborhydrid, Lithiumaluminiumhydrid, Diboran oder Boran/Dimethylsulfid, kann eine vorhandene Carbonyl- oder Nitrilfunktion mitreduziert werden.
d) Umsetzung einer Verbindung der allgemeinen Formel 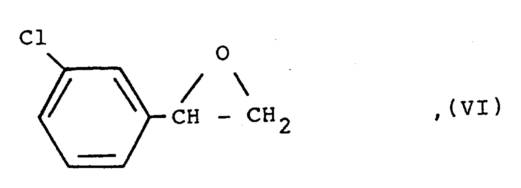 mit einem Amin der allgemeinen Formel 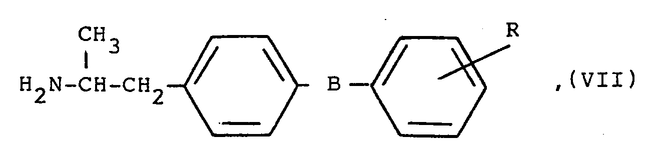 in der
   R und B wie eingangs definiert sind.
   Die Umsetzung wird vorzugsweise in einem Lösungsmittel wie Ethanol, Isopropanol, Tetrahydrofuran, Dioxan, Dimethylformamid, Dimethylsulfoxid oder Acetonitril/Ethanol zweckmäßigerweise bei Temperaturen zwischen 50 und 100°C durchgeführt. Die Umsetzung kann jedoch auch ohne Lösungsmittel durchgeführt werden.
e) Reduktion einer gegebenenfalls im Reaktionsgemisch gebildeten Verbindung der allgemeinen Formel 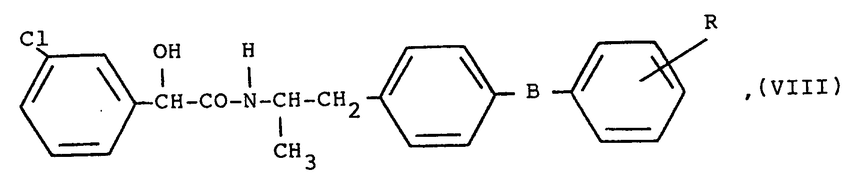 in der
   R und B wie eingangs definiert sind.
   Die Reduktion wird in einem geeigneten Lösungsmittel wie Diethylether oder Tetrahydrofuran mit einem Reduktionsmittel wie einem Metallhydrid, z.B. mit Lithiumaluminiumhydrid, Diboran oder Diboran/Dimethylsulfid, vorzugsweise jedoch mit Natriumborhydrid in Gegenwart von Eisessig oder Trifluoressigsäure, bei Temperaturen zwischen 0 und 100°C, vorzugsweise jedoch bei Temperaturen zwischen 10 und 50°C, durchgeführt.
f) Zur Herstellung von Verbindungen der allgemeinen Formel I, in der R eine 2-Hydroxy-ethoxygruppe darstellt:
   Reduktion einer Verbindung der allgemeinen Formel 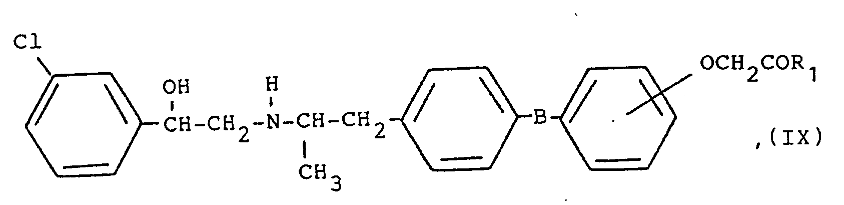 in der
   B wie eingangs definiert ist und
   R₁ eine Hydroxy- oder Alkoxygruppe darstellt.
   Die Reduktion wird in einem geeigneten Lösungsmittel wie Diethylether oder Tetrahydrofuran mit einem Reduktionsmittel wie einem Metallhydrid, z.B. mit Lithiumaluminiumhydrid, Diboran, Diboran/Dimethylsulfid oder mit Natriumborhydrid in Gegenwart von Eisessig oder Trifluoressigsäure, bei Temperaturen zwischen 0 und 100°C, vorzugsweise jedoch bei Temperaturen zwischen 10 und 50°C, durchgeführt. - Bei der Umsetzung kann eine im Rest B enthaltene Carbonylgruppe gleichzeitig mitreduziert werden.
g) Zur Herstellung von Verbindungen der allgemeinen Formel I, in der R eine Methoxycarbonyl-, Ethoxycarbonyl-, Methoxycarbonylmethoxy- oder Ethoxycarbonylmethoxygruppe darstellt:
   Umsetzung einer Verbindung der allgemeinen Formel 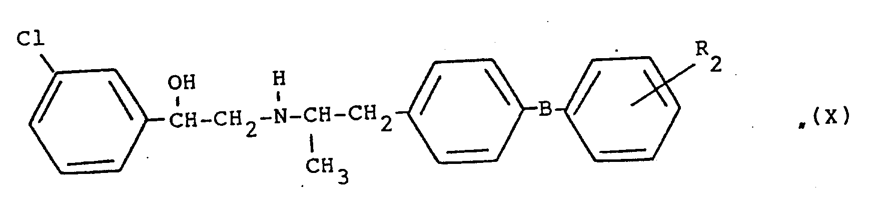 in der
   B wie eingangs definiert ist und
   R₂ eine Carboxy- oder Carboxymethoxygruppe darstellt, oder deren reaktionsfähige Derivate wie beispielsweise deren aktivierte Ester mit einer Verbindung der allgemeinen Formel

   H - R₃ ,(XI)

   in der
   R₃ eine Methoxy- oder Ethoxygruppe darstellt.
   Die Veresterung oder Amidierung wird zweckmäßigerweise in einem Lösungsmittel wie Methylenchlorid, Chloroform, Ether, Tetrahydrofuran, Dioxan, Toluol oder Dimethylformamid, besonders vorteilhaft jedoch in einem Überschuß einer eingesetzten Verbindung der allgemeinen Formel XI, z.B. in Methanol, Ethanol, n-Propanol, Isopropanol, Ammoniak, Methylamin, Ethylamin, Dimethylamin, Diethylamin oder Piperidin gegebenenfalls in Gegenwart eines die Säure aktivierenden Mittels oder eines wasserentziehenden Mittels, z.B. in Gegenwart von Chlorameisensäureäthylester, Thionylchlorid, Phosphortrichlorid, Phosphorpentoxid, N,N'-Dicyclohexylcarbodiimid, N,N'-Dicyclohexylcarbodiimid/N-Hydroxy-succinimid, N,N'-Carbonyldiimidazol oder N,N'-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer anorganischen Base wie Natriumcarbonat oder einer tertiären organischen Base wie Triethylamin oder Pyridin, welche gleichzeitig als Lösungsmittel dienen können, bei Temperaturen zwischen -25°C und 150°C, vorzugsweise jedoch bei Temperaturen zwischen -10°C und der Siedetemperatur des verwendeten Lösungsmittels, durchgeführt.

Bei den vorstehend beschriebenen Verfahren können vorhandene reaktive Gruppen wie Imino- oder Carboxygruppen erforderlichenfalls während der Umsetzung geschützt werden.

Als Schutzgruppen kommen beispielsweise für die Iminogruppe die Acetyl-, Benzoyl-, tert.Butoxycarbonyl-, Benzyloxycarbonyl-, Benzyl-, Trityl- oder Fluorenylmethyloxycarbonylgruppe und
für die Carboxygruppe die Benzyl- oder tert.Butylgruppe in Betracht.

Die gegebenenfalls nach der Umsetzung erforderliche Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser, Dioxan/Wasser oder Eisessig, in Gegenwart einer Säure wie Salzsäure, Schwefelsäure oder Bromwasserstoffsäure oder in Gegenwart einer Alkalibase wie Natriumhydroxid oder Kaliumhydroxid bei Temperaturen zwischen 0 und 150°C, vorzugsweise bei der Siedetemperatur des Reaktionsgemisches. Die Abspaltung eines Benzyl- oder Benzyloxycarbonylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und einem Wasserstoffdruck von 1 bis 7 bar, vorzugsweise jedoch von 3 bis 5 bar.

Erhält man erfindungsgemäß eine Verbindung der allgemeinen Formel I, in der R eine Methoxycarbonyl- oder Ethoxycarbonylgruppe darstellt oder enthält, so kann diese mittels Hydrolyse in eine entsprechende Verbindung der allgemeinen Formel I, in der R eine Carboxygruppe darstellt oder enthält, übergeführt werden.

Die nachträgliche Hydrolyse wird entweder in Gegenwart einer Säure wie Salzsäure, Schwefelsäure oder Trifluoressigsäure oder in Gegenwart einer Base wie Natriumhydroxid oder Kaliumhydroxid in einem geeigneten Lösungsmittel wie Wasser, Methanol, Ethanol, Ethanol/Wasser, Wasser/Isopropanol oder Wasser/Dioxan bei Temperaturen zwischen -10°C und 120°C, z.B. bei Temperaturen zwischen Raumtemperatur und der Siedetemperatur des Reaktionsgemisches, durchgeführt.

Wie bereits eingangs erwähnt, können die neuen Verbindungen in Form ihrer Enantiomeren oder Enantiomerengemische oder, sofern sie mindestens 2 asymmetrische Kohlenstoffatome enthalten, auch in Form ihrer Diastereomeren bzw. Diastereomerengemische vorliegen.

So lassen sich die erhaltenen Verbindungen der allgemeinen Formel I, welche nur ein optisch aktives Zentrum enthalten, nach an sich bekannten Methoden (siehe Allinger N. L. und Elich W. L. in "Topics in Stereochemistry", Vol. 6, Wiley Interscience, 1971) in ihre optischen Antipoden auftrennen, z.B. durch Umkristallisieren aus einem optisch aktiven Lösungsmittel oder durch Umsetzen mit einer, mit der racemischen Verbindung Salze bildenden optisch aktiven Substanz, insbesondere Säuren, und Trennen des auf diese Weise erhaltenen Salzgemisches, z.B. auf Grund von verschiedenen Löslichkeiten, in die diastereomeren Salze, aus denen die freien Antipoden durch Einwirkung geeigneter Mittel freigesetzt werden können. Besonders gebräuchliche, optisch aktive Säuren sind z.B. die D- und L-Formen von Weinsäure, Di-O-Benzoylweinsäure, Äpfelsäure, Mandelsäure, Camphersulfonsäure, Glutaminsäure, Asparaginsäure oder Chinasäure.

Desweiteren lassen sich die erhaltenen Verbindungen der allgemeinen Formel I mit mindestens 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen. Ein so erhaltenes Enantiomerenpaar läßt sich anschließend in seine optischen Antipoden, wie oben beschrieben, auftrennen. Enthält beispielsweise eine Verbindung der allgemeinen Formel I zwei optisch aktive Kohlenstoffatome, so erhält man die entsprechenden (R R', S S')- und (R S', S R')-Formen.

Desweiteren können die erhaltenen Verbindungen der allgemeinen Formel I in ihre Additionssalze, insbesondere für die pharmazeutische Anwendung, in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Essigsäure, Oxalsäure, Malonsäure, Fumarsäure, Maleinsäure, Bernsteinsäure, Ascorbinsäure, Äpfelsäure, Weinsäure, Milchsäure, Zitronensäure, Benzoesäure, Methansulfonsäure, Toluolsulfonsäure, Phthalsäure, Naphthalinsulfonsäure, Nikotinsäure, Palmitinsäure oder Embonsäure in Betracht.

Die als Ausgangsstoffe verwendeten Verbindungen der allgemeinen Formeln II bis XI erhält man nach literaturbekannten Verfahren bzw. sind literaturbekannt.

So erhält man beispielsweise eine Ausgangsverbindung der allgemeinen Formel II durch Friedel-Crafts-Acetylierung einer entsprechenden Verbindung, anschließende Bromierung und gegebenenfalls anschließende Umsetzung mit Urotropin und nachfolgende Hydrolyse. Ein so erhaltenes Keton wird anschließend reduziert.

Eine Ausgangsverbindung der allgemeinen Formel IV erhält man durch Umsetzung einer entsprechenden Carbonylverbindung mit einem entsprechenden Amin.

Eine Ausgangsverbindung der allgemeinen Formel V erhält man durch Umsetzung einer entsprechenden Halogenacetyl- oder Glyoxalverbindung mit einem entsprechenden Amin.

Eine Ausgangsverbindung der allgemeinen Formel VIII erhält man durch Umsetzung einer entsprechenden Carbonsäure mit einem entsprechenden Amin in Gegenwart eines die Säure aktivierenden Mittels.

Eine Ausgangsverbindung der allgemeinen Formeln IX und X erhält man durch Umsetzung eines entsprechenden ω-Halogenalkohols mit einem entsprechenden Amin.

Wie bereits eingangs erwähnt, weisen die neuen Verbindungen der allgemeinen Formel I, deren Enantiomere oder Enantiomerengemische oder, sofern sie mindestens 2 asymmetrische Kohlenstoffatome enthalten, auch deren Diastereomere bzw. Diastereomerengemische und deren Additionssalze, insbesondere für die pharmazeutische Anwendung deren physiologisch verträgliche Säureadditionssalze, wertvolle pharmakologische Eigenschaften auf, insbesondere eine Wirkung auf den Stoffwechsel, vorzugsweise eine blutzuckersenkende, körperfettreduzierende und Energieverbrauch-steigernde Wirkung, sowie eine Senkung der atherogenen Lipoproteine VLDL und LDL.

Beispielsweise wurden die nachfolgenden Verbindungen auf ihre biologischen Eigenschaften wie folgt untersucht:
- A =: 4'-[2-[N-(2-(3-Chlor-phenyl)-2-hydroxy-ethyl)amino]propyl]biphenylyl-4-carbonsäure-ethylester,
- B =: 4'-[2-[N-(2-(3-Chlor-phenyl)-2-hydroxy-ethyl)amino]propyl]biphenylyl-4-oxyessigsäure-methylester,
- C =: 4'-[2-[N-(2-(3-Chlor-phenyl)-2-hydroxy-ethyl)amino]propyl]-4-(2-hydroxy-ethoxy)biphenyl und
- D =: 4'-[2-[N-(2-(3-Chlor-phenyl)-2-hydroxy-ethyl)amino]propyl]diphenylmethan-2-carbonsäure-ethylester.

### 1. Antidiabetische Wirkung:

Die antidiabetische Wirkung der erfindungsgemäßen Verbindungen läßt sich als eine blutzuckersenkende Wirkung bei Versuchstieren messen. Die zu untersuchenden Substanzen wurden dazu in 1,5%iger Methylzellulose suspendiert und weiblichen Mäusen eigener Zucht mittels Schlundsonde appliziert. 30 Minuten später wurde 1 g Glukose pro kg Körpergewicht in Wasser gelöst und subkutan appliziert. Weitere 30 Minuten später wurde aus dem retroorbitalen Venenplexus Blut entnommen. Aus dem Serum wurde Glukose mit der Hexokinase-Methode mit Hilfe eines Analysenphotometers bestimmt.

In der nachstehenden Tabelle sind die bei dieser Versuchsanordnung beobachteten Blutzuckersenkungen in % einer mitgeführten Kontrollgruppe zusammengestellt. Die statistische Auswertung erfolgte nach dem t-Test nach Student mit p=0,05 als Signifikanzgrenze.

| Verbindung | % Änderung vom Wert der Kontrollgruppe Dosis [mg/kg] | |
|---|---|---|
| | 1 | 3 |
| A | - 37 | - 49 |
| B | - 27 | - 44 |
| C | | - 62 |
| D | | - 62 |

### 2. Antiadipöse Wirkung:

Die antiadipöse Wirkung der erfindungsgemäßen Verbindungen, die sich in einer Steigerung der Lipolyse ausdrückt, wurde am Anstieg des Glyzerins im Serum gemessen. Der Versuchsablauf ist identisch mit der zur Testung auf blutzuckersenkende Wirkung vorstehend beschriebenen Versuchsanordnung. Glyzerin wurde in einem kombinierten enzymatisch-kolorimetrischen Test mit Hilfe eines Analysenphotometers bestimmt. Die Ergebnisse sind in der nachfolgenden Tabelle in % einer mitgeführten Kontrollgruppe aufgeführt.

| Verbindung | % Änderung vom Wert der Kontrollgruppe Dosis [mg/kg] | |
|---|---|---|
| | 1 | 3 |
| A | 121 | 220 |
| B | | 185 |
| C | | 378 |
| D | | 402 |

Desweiteren konnten bei den vorstehend beschriebenen Untersuchungen der erfindungsgemäßen Substanzen bei den applizierten Dosen keine Kreislaufwirkungen und bis zu einer Dosis von 30 mg/kg keine toxischen Nebenwirkungen beobachtet werden. Diese sind daher gut verträglich.

Auf Grund ihrer pharmakologischen Eigenschaften eignen sich daher die neuen Verbindungen der allgemeinen Formel I sowie deren physiologisch verträgliche Additionssalze mit anorganischen oder organischer Säuren zur Behandlung sowohl des Diabetes mellitus als auch der Adipositas, insbesondere also zur Behandlung des adipösen Diabetikers. Hierbei kann die erforderliche Dosis ganz auf den stoffwechselphysiologischen Bedarf des einzelnen Patienten abgestimmt werden, da die Substanzen über einen großen Dosisbereich frei von einer Herz/Kreislaufwirkung sind. Beim Erwachsenen liegen daher die Tagesdosen zwischen 1 und 300 mg, vorzugsweise jedoch bei 1 bis 100 mg, verteilt auf 1 bis 4 Dosen pro Tag. Hierzu lassen sich die obenerwähnten Verbindungen, gegebenenfalls in Kombination mit anderen Wirksubstanzen, in die üblichen galenischen Zubereitungsformen wie Pulver, Tabletten, Dragees, Kapseln, Suppositorien oder Suspensionen einarbeiten.

Die vorstehend erwähnten Verbindungen können ferner als Folge ihrer körperfettreduzierenden (lipolytischen) Wirkung zur Behandlung fettsüchtiger Tiere und zur Reduktion oder Verhinderung unerwünschten Fettansatzes bei der Mast von Tieren und damit zur Verbesserung der Fleischqualität von Masttieren eingesetzt werden. Weiterhin können die genannten Verbindungen zur Erzielung höherer täglicher Gewichtszunahmen und verbesserter Futterausnutzung in der Tierernährung, vorzugsweise bei der Mast von Tieren, verwendet werden.

Die leistungsfördernde und fettreduzierende Wirkung der vorstehend erwähnten Verbindungen der Formel I wurde beispielshaft an den Substanzen A, B und D wie folgt geprüft:
1.) 4 Gruppen von 20 (Kontrolle) bzw. 10 (Versuchsgruppen A, B und D) männlichen Mäusen, wobei jedes Tier einzeln gehalten wurde, erhielten identisches Futter und Wasser ad libitium. Die Versuchsgruppen erhielten zusätzlich 20 ppm der vorstehend erwähnten Verbindungen (A, B und D) über das Futter verabreicht. Die Versuchsperiode dauerte 14 Tage.
Die nachfolgende Tabelle enthält die gefundenen Ergebnisse als relative Veränderungen gegenüber Kontrolle (Kontrolle = 100):

| | A | B | D |
|---|---|---|---|
| Anfangsgewicht | 101,2 | 100,3 | 99,3 |
| Gewichtszunahme | 90,5 | 183,2 | 239,5 |
| Futteraufnahme | 91,6 | 106,0 | 106,5 |
| Gewichtszunahme/Futteraufnahme | 99,1 | 173,0 | 225,1 |

2.) 4 Gruppen von 20 (Kontrolle) bzw. 10 (Versuchsgruppen A, B und D) männlichen Mäusen, wobei jedes Tier einzeln gehalten wurde, erhielten identisches Futter und Wasser ad libitum. Die Versuchsgruppen erhielten zusätzlich 10 mg/kg Körpergewicht der vorstehend erwähnten Verbindungen p.o.. Die Versuchsperiode dauerte 4 Tage.
Die nachfolgende Tabelle enthält die gefundenen Ergebnisse als relative Veränderungen gegenüber Kontrolle (Kontrolle = 100):

| | A | B | D |
|---|---|---|---|
| Gewichtszunahme/Futteraufnahme | 90,5 | 122,5 | 54,5 |
| Nebenhodenfettdepot | 89,6 | 97,7 | 74,2 |
| Musculus gastrocnemius | 111,4 | 103,3 | 110,7 |
| Musculus soleus | 115,1 | 120,2 | 108,7 |
| Fleisch : Fett-Verhältnis | 126,0 | 112,2 | 147,0 |

Auf Grund dieser Eigenschaften können die vorstehend erwähnten Wirkstoffe als Leistungsförderer bei Tieren zur Förderung und Beschleunigung des Wachstums, der Milch- und Wollproduktion, sowie zur Verbesserung der Futterverwertung, der Schlachtkörperqualität und zur Verschiebung des Fleisch-Fett-Verhältnisses zugunsten von Fleisch eingesetzt werden. Die Wirkstoffe werden bei Nutz-, Zucht-, Zier- und Hobbytieren verwendet.

Zu den Nutz- und Zuchttieren zählen Säugetiere, z.B. Rinder wie Kälber, Ochsen, Bullen, Färsen und Kühe, Büffel, Schweine, Pferde, Schafe, Ziegen, Kaninchen, Hasen, Damwild, Pelztiere wie Nerze und Chinchilla, Geflügel wie Tauben, Hühner, Gänse, Enten, Truthühner, Perlhühner, Fasanen und Wachteln, Fische, wie Karpfen, Forellen, Lachse, Aale, Schleien und Hechte, oder Reptilien wie Schlangen und Krokodile.

Zu den Zier- und Hobbytieren zählen Säugetiere wie Hunde und Katzen, Vögel wie Papageien, Kanarienvögel, Fische wie Zier- und Aquarienfische, z.B. Goldfische.

Die Wirkstoffe werden unabhängig vom Geschlecht der Tiere während allen Wachstums- und Leistungsphasen der Tiere eingesetzt. Bevorzugt werden die Wirkstoffe während der intensiven Wachstums- und Leistungsphase eingesetzt. Die intensive Wachstums- und Leistungsphase dauert je nach Tierart von einem Monat bis zu 10 Jahren.

Die Menge der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,001 bis 50 mg/kg insbesondere 0,01 bis 10 mg/kg Körpergewicht pro Tag. Die passende Menge des Wirkstoffs sowie die passende Dauer der Verabreichung hängen insbesondere von der Tierart, dem Alter, dem Geschlecht, dem Gesundheitszustand und der Art der Haltung und Fütterung der Tiere ab und sind durch jeden Fachmann leicht zu ermitteln.

Die Wirkstoffe werden den Tieren nach den üblichen Methoden verabreicht. Die Art der Verabreichung hängt insbesondere von der Tierart, dem Verhalten und dem Gesundheitszustand der Tiere ab.

Die Wirkstoffe können einmalig verabreicht werden. Die Wirkstoffe können aber auch während der ganzen oder während eines Teils der Wachstumsphase temporär oder kontinuierlich verabreicht werden. Bei kontinuierlicher Verabreichung kann die Anwendung ein- oder mehrmals täglich in regelmäßigen oder unregelmäßigen Abständen erfolgen.

Die Verabreichung erfolgt oral oder parenteral in dafür geeigneten Formulierungen oder in reiner Form. Orale Formulierungen sind Pulver, Tabletten, Granulate, Drenche, Boli sowie Futtermittel, Prämixe für Futtermittel, Formulierungen zur Verabreichung über Trinkwasser.

Die oralen Formulierungen enthalten den Wirkstoff in Konzentrationen von 0,01 ppm - 100 %, bevorzugt von 0,01 ppm - 10 %.

Parenterale Formulierungen sind Injektionen in Form von Lösungen, Emulsionen und Suspensionen, sowie Implantate.

Die Wirkstoffe können in den Formulierungen allein oder in Mischung mit anderen Wirkstoffen, Mineralsalzen, Spurenelementen, Vitaminen, Eiweißstoffen, Farbstoffen, Fetten oder Geschmacksstoffen vorliegen.

Die Konzentration der Wirkstoffe im Futter beträgt normalerweise etwa 0,01 - 500 ppm, bevorzugt 0,1 - 50 ppm.

Die Wirkstoffe können als solche oder in Form von Prämixen oder Futterkonzentraten dem Futter zugesetzt werden.

So enthalten die erfindungsgemäßen Futtermittel neben dem Wirkstoff und gegebenenfalls neben einer üblichen Vitamin-Mineral-Mischung beispielsweise für Mastschweine Gerste, Weizennachmehl, Ackerbohnen, Raps-Extraktionsschrot und Futterfett, für Broiler Mais, Sojabohnenmehl, Fleischmehl, Futterfett und Sojaöl, für Rinder Zuckerrübenschnitzel Maiskleber, Malzkeime, Sojabohnenmehl, Weizen und Molasse sowie für Lämmer Gerste, Sojabohnenmehl, Mais und Melasse. Zu diesem Futter wird eine der eingangs erwähnten Verbindungen der Formel I als Wirkstoff in einer Konzentration von 0,01 bis 500 ppm, vorzugsweise jedoch von 0,1 bis 50 ppm, zugemischt, wobei die Zumischung vorzugsweise in Form einer Wirkstoffvormischung erfolgt. Diese Vormischung enthält beispielsweise pro 10 g 10 mg Wirkstoff zweckmäßigerweise in 9,99 g Maisstärke.

Die nachfolgenden Beispiele sollen die Erfindung näher erläutern:

### Herstellung der Ausgangsverbindungen

### Beispiel A

### 4'-Acetonyl-biphenylyl-4-carbonsäure-ethylester

a) 4'-(2-Chlor-propionyl)biphenylyl-4-carbonsäure-ethylester
   Eine Lösung von 10 g (0,044 Mol) Biphenylyl-4-carbonsäure-ethylester in 70 ml Methylenchlorid tropft man bei 0°C zu einer Lösung von 25 g (0,187 Mol) Aluminiumchlorid und 9,6 g (0,076 Mol) 2-Chlor-propionylchlorid in 250 ml Methylenchlorid. Nach Stehen über Nacht bei Raumtemperatur gibt man auf Eiswasser und verdünnte Salzsäure und extrahiert mit Methylenchlorid. Die Extrakte werden getrocknet und nach dem Einengen im Fließmittel Cyclohexan/Essigsäure-ethylester (7:1) säulenchromatographisch gereinigt. Dabei werden 6,9 g 4'-(2-Chlor-propionyl)biphenylyl-4-carbonsäure-ethylester erhalten.
   Schmelzpunkt: 75-78°C
b) 6,8 g (0,021 Mol) 4'-(2-Chlor-propionyl)biphenylyl-4-carbonsäure-ethylester werden in 40 ml Aceton mit 3,9 g (0,034 Mol) Kaliumacetat 2 Tage am Rückfluß gekocht. Anschließend wird vom Niederschlag abfiltriert, das Filtrat eingeengt und der Einengungsrückstand säulenchromatographisch gereinigt (Fließmittel: Cyclohexan/Essigsäure-ethylester = 7:1). Hierbei werden 5,5 g 4'-(2-Acetoxy-propionyl)-biphenylyl-4-carbonsäure-ethylester erhalten.
c) 5,5 g (0,016 Mol) 4'-(2-Acetoxy-propionyl)-biphenylyl-4-carbonsäure-ethylester werden in 160 ml Ethanol gelöst und bei 5-10°C portionsweise mit Natriumborhydrid versetzt (ca. 1 g) bis im Chromatogramm kein Ausgangsmaterial mehr nachweisbar ist. Anschließend wird eingeengt, mit Wasser zersetzt und mit Essigsäure-ethylester extrahiert. Die Extrakte werden eingeengt und nach Zugabe von 50 g Polyphosphorsäure 30 Minuten im Ölbad bei 80°C gerührt. Danach gibt man auf Eiswasser und extrahiert mit Essigsäure-ethylester. Die Extrakte werden getrocknet, eingeengt und an Kieselgel säulenchromatographisch gereinigt (Fließmittel: Cyclohexan/Essigsäure-ethylester = 3:1).
   Ausbeute: 2,8 g (62 % der Theorie)
   Schmelzpunkt: 71-73°C
   Analog Beispiel A werden erhalten:
   4'-Acetonyl-4-methoxy-biphenyl,
   Schmelzpunkt: 89-92°C
   4'-Acetonyl-2-methoxy-biphenyl,
   Schmelzpunkt: <20°C
   4'-Acetonyl-biphenylyl-2-carbonsäure-ethylester,
   Schmelzpunkt: <20°C
   4'-Acetonyl-diphenylmethan-2-carbonsäure-ethylester.

### Beispiel B

### 4'-Acetonyl-4-hydroxy-biphenyl

4,8 g (20 mMol) 4'-Acetonyl-4-methoxy-biphenyl werden in 50 ml Benzol gelöst und nach Zugabe von 6,14 g (46 mMol) Aluminiumchlorid 24 Stunden am Rückfluß gekocht. Dann gibt man auf Eis und verdünnte Salzsäure, extrahiert mit Chloroform und reinigt die Extrakte durch Kieselgelchromatographie (Fließmittel: Toluol/Essigsäure-ethylester = 6:1).
Ausbeute: 2,8 g (62 % der Theorie),
Schmelzpunkt: 151-153°C
Analog Beispiel B wird erhalten:
4'-Acetonyl-2-hydroxy-biphenyl

### Beispiel C

### 4'-Acetonyl-biphenylyl-2-oxyessigsäure-methylester

4,3 g (19 mMol) 4'-Acetonyl-2-hydroxy-biphenyl werden in 50 ml Aceton gelöst und nach Zugabe von 2,63 g (19 mMol) Kaliumcarbonat und 1,8 ml (19 mMol) Bromessigsäure-methylester 5 Stunden am Rückfluß gekocht. Anschließend wird filtriert, das Filtrat eingeengt und der Rückstand an Kieselgel chromatographiert (Fließmittel: Toluol/Essigsäure-ethylester = 10:1).
Ausbeute: 5,0 g (88,2 % der Theorie),
Schmelzpunkt: <20°C
Analog Beispiel C wird erhalten:
4'-Acetonyl-biphenylyl-4-oxyessigsäure-methylester,
Schmelzpunkt: 81-82°C

### Beispiel 1

### 4'-[2-[N-(2-(3-Chlor-phenyl)-2-hydroxy-ethyl)amino]propyl]-biphenylyl-4-carbonsäure-ethylester

5,6 g (20 mMol) 4'-Acetonyl-biphenylyl-4-carbonsäure-ethyl-ester und 3,4 g (20 mMol) 2-(3-Chlor-phenyl)-2-hydroxy-ethyl-amin werden in 75 ml Ethanol gelöst und nach Zugabe von 1,25 g (20 mMol) Natriumcyanoborhydrid und 1,2 ml (20 mMol) Eisessig 24 Stunden bei Raumtemperatur gerührt. Anschließend wird eingeengt, auf Wasser gegeben und mit verdünnter Salzsäure angesäuert. Nach 30 Minuten Rühren wird mit Natronlauge alkalisch gestellt und mit Essigsäure-ethylester extrahiert. Die Extrakte werden eingeengt und der Einengungsrückstand an Kieselgel säulenchromatographisch gereinigt (Fließmittel: Essigsäure-ethylester/Methanol = 40:1). Anschließend wird aus Acetonitril umkristallisiert.
Ausbeute: 4,3 g (49 % der Theorie),
Schmelzpunkt: 104-116°C

| | | | | | | | | |
|---|---|---|---|---|---|---|---|---|
| Ber.: | C | 71,30 | H | 6,44 | N | 3,20 | Cl | 8,10 |
| Gef.: | | 71,45 | | 6,30 | | 3,47 | | 8,08 |

Laut ¹H-NMR (400 MHZ) liegt ein 1:1 Diastereomerenpaargemisch vor.
[CDCl₃: δ = 4,56 ppm (dd, 0,5 H), δ = 4,62 ppm (dd, 0,5 H)]
Analog Beispiel 1 werden folgende Verbindungen hergestellt:
a) 4'-[2-[N-(2-(3-Chlor-phenyl)-2-hydroxy-ethyl)amino]propyl]biphenylyl-4-oxyessigsäure-methylester
Hergestellt aus 2-(3-Chlor-phenyl)-2-hydroxy-ethylamin und 4'-Acetonyl-biphenylyl-4-oxyessigsäure-methylester.
Ausbeute: 25 % der Theorie,
Schmelzpunkt: 106-109°C

| | | | | | | | | |
|---|---|---|---|---|---|---|---|---|
| Ber.: | C | 68,79 | H | 6,22 | N | 3,09 | Cl | 7,81 |
| Gef.: | | 68,90 | | 6,06 | | 3,07 | | 7,62 |

Laut ¹H-NMR (400 MHZ) liegt ein 1:1 Diastereomerenpaargemisch vor.
[CDCl₃: δ = 4,55 ppm (dd), δ = 4,615 ppm (dd)]
b) 4'-[2-[N-(2-(3-Chlor-phenyl)-2-hydroxy-ethyl)amino]propyl]-biphenylyl-2-carbonsäure-ethylester
Hergestellt aus 2-(3-Chlor-phenyl)-2-hydroxy-ethylamin und 4'-Acetonyl-biphenylyl-2-carbonsäure-ethylester.
Ausbeute: 39,3 % der Theorie,
Schmelzpunkt: <20°C

| | | | | | | |
|---|---|---|---|---|---|---|
| Ber.: | C | 71,30 | H | 6,44 | N | 3,20 |
| Gef.: | | 71,55 | | 6,27 | | 3,09 |

Laut ¹H-NMR (400 MHZ) liegt ein 1:1 Diastereomerenpaargemisch vor.
[CDCl₃: δ = 4,50 ppm (dd, 0,5 H), δ = 4,60 ppm (dd, 0,5 H)]
c) 4'-[2-[N-(2-(3-Chlor-phenyl)-2-hydroxy-ethyl)amino]propyl]-biphenylyl-2-oxyessigsäure-methylester
Hergestellt aus 2-(3-Chlor-phenyl)-2-hydroxy-ethylamin und 4'-Acetonyl-biphenylyl-2-oxyessigsäure-methylester.
Ausbeute: 83 % der Theorie,
Schmelzpunkt: <20°C

| | | | | | | |
|---|---|---|---|---|---|---|
| Ber.: | C | 68,79 | H | 6,22 | N | 3,08 |
| Gef.: | | 68,80 | | 6,10 | | 2,91 |

Laut ¹H-NMR (400 MHZ) liegt ein 1:1 Diastereomerenpaargemisch vor.
[CDCl₃: δ = 4,686 ppm (dd, 0,5 H), δ = 4,62 ppm (dd, 0,5 H)]
d) 4'-[2-[N-(2-(3-Chlor-pnenyl)-2-hydroxy-ethyl)amino]propyl]diphenylmethan-2-carbonsäure-ethylester
Hergestellt aus 2-(3-Chlor-phenyl)2-hydroxy-ethylamin und 4'-Acetonyl-diphenylmethan-2-carbonsäure-ethylester.
Ausbeute: 83 % der Theorie,
Schmelzpunkt: <20°C

| | | | | | | | | |
|---|---|---|---|---|---|---|---|---|
| Ber.: | C | 71,75 | H | 6,69 | N | 3,10 | Cl | 7,84 |
| Gef .: | | 71,45 | | 6,87 | | 2,94 | | 8,03 |

Laut ¹H-NMR (400 MHZ) liegt ein 1:1 Diastereomerenpaargemisch vor.
[CDCl₃: δ = 4,51 ppm (dd, 0,5 H), δ = 4,59 ppm (dd, 0,5 H)]

### Beispiel 2

### 4'-[2-(N-(2-(3-Chlor-phenyl)-2-hydroxy-ethyl)amino]propyl]-biphenylyl-4-carbonsäure-ethylester

### a) 4-(2-Phtalimido-pronyl)-biphenyl

9,0 g (0,0426 Mol) 4-(2-Amino-propyl)biphenyl werden in 50 ml Glykol gelöst und nach Zugabe von 6,3 g (0,0426 Mol) Phthalsäureanhydrid 2 Stunden bei 170°C gerührt. Dann wird mit Wasser verdünnt und das 4-(2-Phthalimido-propyl)biphenyl mit Essigsäure-ethylester extrahiert. Nach dem Einengen der Extrakte und Kristallisation aus Methanol erhält man 10,4 g (72 % der Theorie) vom Schmelzpunkt 143-145°C.

### b) 4'-(2-Phthalimido-propyl)-4-acetyl-biphenyl

3 g (8,8 mMol) 4-(2-Phthalimido-propyl)biphenyl werden in 50 ml Schwefelkohlenstoff vorgelegt und nach Zugabe von 4 g (0,03 Mol) Aluminiumchlorid tropfenweise mit 0,78 g (0,01 Mol) Acetylchlorid versetzt. Nach Stehenlassen über Nacht gibt man auf Eiswasser/verdünnte Salzsäure und extrahiert mit Chloroform. Durch Einengen der Extrakte und Kristallisation aus Aceton erhält man 1,58 g (47 % der Theorie) 4'-(2-Phthalimido-propyl)-4-acetyl-biphenyl vom Schmelzpunkt 157-159°C.

### c) 4'-(2-Phthalimido-propyl)-biphenylyl-4-carbonsäure

0,9 g (2,3 mMol) 4'-(2-Phthalimido-propyl)-4-acetyl-biphenyl werden in 10 ml Dioxan und 10 ml Wasser suspendiert und nach Zugabe von 0,96 g (24 mMol) gepulvertem Natriumhydroxid unter Kühlung tropfenweise mit 1,43 g (9 mMol) Brom versetzt. Man rührt eine Stunde nach, säuert dann mit Salzsäure an und saugt vom Niederschlag ab. Man erhält 0,8 g (90,3 % der Theorie) an 4'-(2-Phthalimido-propyl)biphenylyl-4-carbonsäure vom Schmelzpunkt 280°C.

### d) 4'-(2-Amino-propyl)biphenylyl-4-carbonsäure-ethylester

2,0 g (5,2 mMol) 4'-(2-Phthalimido-propyl)-biphenylyl-4-carbonsäure werden in 50 ml konzentrierter Salzsäure suspendiert und im Druckgefäß bei 120°C 6 Stunden geschüttelt. Dann wird zur Trockene eingeengt, mit warmem Aceton digeriert und vom Niederschlag abgesaugt. Der Niederschlag wird in Ethanol gelöst und unter Einleiten von trockenem Salzsäuregas 2 Stunden am Rückfluß gekocht. Anschließend wird eingeengt, alkalisch gestellt und mit Essigsäure-ethylester extrahiert. Nach dem Einengen der Extrakte werden 0,6 g 4'-(2-Amino-propyl)biphenylyl-4-carbonsäure-ethylester erhalten (41 % der Theorie).

### e) 4'-[2-[N(2-(3-Chlor-phenyl)-2-hydroxy-ethyl)amino]propyl]-biphenylyl-4-carbonsäure-ethylester

1,0 g (4,59 mMol) 3-Chlor-phenacylbromid werden in 20 ml Methylenchlorid gelöst und tropfenweise zu einer Lösung von 1,3 g (4,59 mMol) 4'-(2-Amino-propyl)biphenylyl-4-carbonsäure-ethylester und 0,64 g (5 mMol) N,N-Diisopropyl-ethylamin in 100 ml Methylenchlorid bei Raumtemperatur gegeben. Nach 2 Stunden werden 10 ml Methanol zugegeben und unter leichter Kühlung 0,23 g (6 mMol) Natriumborhydrid eingetragen. Nach Rühren über Nacht wird im Vakuum eingeengt, mit verdünnter Salzsäure angesäuert und 30 Minuten gerührt. Anschließend stellt man mit Natronlauge alkalisch und extrahiert mit Chloroform. Der Chloroform-Einengungsrückstand wird an Kieselgel säulenchromatographisch gereinigt (Fließmittel: Chloroform/Methanol = 10:1). Zuletzt wird aus Acetonitril umkristallisiert.
Ausbeute: 0,5 g (25 % der Theorie),
Schmelzpunkt: 94-96°C

### Beispiel 3

### 4'-[2-[N-(2-(3-Chlor-phenyl)-2-hydroxy-ethyl)amino]propyl]-biphenylyl-4-carbonsäure

0,5 g (1,1 mMol) 4'-[2-[N-(2-(3-Chlor-phenyl)-2-hydroxyethyl)amino]propyl]biphenylyl-4-carbonsäure-ethylester werden in 30 ml Ethanol suspendiert und nach Zugabe von 2 ml 2 N Natronlauge 5 Stunden bei 50°C gerührt. Anschließend wird mit 4 ml 1 N Salzsäure neutralisiert, filtriert und das Filtrat mit 40 ml Wasser verdünnt. Nach Stehen über Nacht wird vom gebildeten Kristallisat abgesaugt und mit Ethanol/ Wasser = 1:1 nachgewaschen.
Ausbeute: 360 mg (80 % der Theorie),
Schmelzpunkt: 199-201°C

| | | | | | | | | |
|---|---|---|---|---|---|---|---|---|
| Ber.: | C | 70,32 | H | 5,90 | N | 3,42 | Cl | 8,65 |
| Gef.: | | 70,22 | | 5,97 | | 3,38 | | 8,60 |

Laut ¹H-NMR (400 MHZ) liegt ein 1:1 Diastereomerenpaargemisch vor.
[DMSO-d₆/CD₃OD: δ = 4,98 ppm (dd), δ = 5,01 ppm (dd)]
Analog Beispiel 3 wird folgende Verbindung hergestellt:
4'-[2-[N-(2-(3-Chlor-phenyl)-2-hydroxy-ethyl)amino]propyl]-biphenylyl-4-oxyessigsäure
Hergestellt aus 4'-[2-[N-(2-(3-Chlor-phenyl)-2-hydroxyethyl)-amino propyl]biphenylyl-4-oxyessigsäure-ethylester Ausbeute: 95,5 % der Theorie,
Schmelzpunkt: 216-218°C

| | | | | | | |
|---|---|---|---|---|---|---|
| Ber.: | C | 68,25 | H | 5,96 | N | 3,18 |
| Gef.: | | 68,37 | | 5,97 | | 3,26 |

Laut ¹H-NMR (400 MHZ) liegt ein 1:1 Diastereomerenpaargemisch vor.

### Beispiel 4

### 4'-[2-[N-(2-(3-Chlor-phenyl)-2-hydroxy-ethyl)-amino]propyl]-4-(2-hydroxy-ethoxy)biphenyl

Zu 1,75 g (46 mMol) Lithiumaluminiumhydrid in 40 ml absolutem Tetrahydrofuran tropft man unter Rühren eine Lösung von 1,75 g (3,85 mMol) 4'-[2-[N-(2-(3-Chlor-phenyl)-2-hydroxyethyl)amino]propyl]biphenylyl-4-oxyessigsäure-methylester in 30 ml absolutem Tetrahydrofuran. Anschließend erwärmt man eine Stunde am Rückfluß, zersetzt dann durch tropfenweise Zugabe von 4N Natronlauge und filtriert vom gebildeten Natriumaluminat ab. Das Filtrat wird eingeengt und der Rückstand aus Acetonitril umkristallisiert.
Ausbeute: 660 mg (40,2 % der Theorie),
Schmelzpunkt: 120-126°C

| | | | | | | |
|---|---|---|---|---|---|---|
| Ber.: | C | 70,49 | H | 6,63 | N | 3,29 |
| Gef.: | | 70,50 | | 6,76 | | 3,42 |

Laut ¹H-NMR (400 MHZ) liegt ein 1:1 Diastereomerenpaargemisch vor.
[CDCl₃: δ = 4,56 ppm (dd, 0,5 H), δ = 4,62 ppm (dd, 0,5 H)]

### Beispiel 5

### Dragée mit 10 mg 4'-[2-[N-(2-(3-Chlor-phenyl)-2-hydroxyethyl)amino]propyl]diphenylmethan-2-carbonsäure-ethylester

### Zusammensetzung:

### 1 Dragée enthält:

| | |
|---|---|
| (1) Wirksubstanz | 10,0 mg |
| (2) Milchzucker | 69,0 mg |
| (3) Maisstärke | 35,0 mg |
| (4) Polyvinylpyrrolidon | 5,0 mg |
| (5) Magnesiumstearat | 1,0 mg |
| | 1̅2̅0̅,̅0̅ m̅g̅ |

### Herstellung:

(1) + (2) + (3) werden gemischt und mit (4) in einer wäßrigen Lösung befeuchtet. Die feuchte Masse wird durch ein Sieb mit 1,6 mm Maschenweite geschlagen und bei 45°C im Umlufttrockenschrank getrocknet. Das trockene Granulat wird durch ein Sieb mit 1 mm Maschenweite gegeben und mit (5) gemischt. Die fertige Mischung wird zu Dragéekernen verpreßt.

| | |
|---|---|
| Kerngewicht: | 120,0 mg |
| Durchmesser: | 7,0 mm |
| Wölbungsradius: | 6,0 mm |

Die so hergestellten Dragéekerne werden auf bekannte Weise mit einer Schicht überzogen, die im Wesentlichen aus Zucker und Talkum besteht. Diese Schicht kann auch Farbauszüge enthalten. Die fertigen Dragees werden mit Wachs poliert.

| | |
|---|---|
| Dragéegewicht: | 180,0 mg |

### Beispiel 6

### Dragée mit 50 mg 4'-[2-[N-(2-(3-Chlor-phenyl)-2-hydroxyethyl)amino]propyl]-diphenylmethan-2-carbonsäure-ethylester

### Zusammensetzung:

### 1 Dragée enthält:

| | |
|---|---|
| (1) Wirksubstanz | 50,0 mg |
| (2) Milchzucker | 110,8 mg |
| (3) Maisstärke | 50,0 mg |
| (4) Polyvinylpyrrolidon | 8,0 mg |
| (5) Magnesiumstearat | 1,2 mg |
| | 2̅2̅0̅,̅0̅ m̅g̅ |

### Herstellung:

Die Herstellung erfolgt analog Beispiel I.

| | |
|---|---|
| Kerngewicht: | 220,0 mg |
| Durchmesser: | 9,0 mm |
| Wölbungsradius: | 8,0 mm |
| Dragéegewicht: | 300,0 mg |

### Beispiel 7

### Tabletten mit 150 mg 4'-[2-[N-(2-(3-Chlor-phenyl)-2-hydroxyethyl)amino]propyl]-diphenylmethan-2-carbonsäure-ethylester

### Zusammensetzung:

### 1 Tablette enthält:

| | |
|---|---|
| (1) Wirksubstanz | 150,0 mg |
| (2) Milchzucker | 86,0 mg |
| (3) Maisstärke | 50,8 mg |
| (4) Mikrokristalline Zellulose | 25,0 mg |
| (5) Polyvinylpyrrolidon | 7,0 mg |
| (6) Magnesiumstearat | 1,2 mg |
| | 3̅2̅0̅,̅0̅ m̅g̅ |

### Herstellung:

(1) + (2) + (3) + (4) + (5) werden gemischt und mit Wasser befeuchtet. Die feuchte Masse wird durch ein Sieb mit 1,6 mm Maschenweite geschlagen und bei 45°C getrocknet. Das trockene Granulat wird nochmals durch dasselbe Sieb gegeben und mit (6) gemischt. Aus der fertigen Mischung werden Tabletten gepreßt.

| | |
|---|---|
| Tablettengewicht: | 320,0 mg |
| Durchmesser: | 10,0 mm |

Die Tabletten werden mit einer Teilkerbe versehen, um die Halbierung zu ermöglichen.

### Beispiel 8

### Hartgelatinekapseln zu 100 mg 4'-[2-[N-(2-(3-Chlor-phenyl)-2-hydroxy-ethyl)amino]propyl]-diphenylmethan-2-carbonsäure-ethylester

### Zusammensetzung:

### 1 Kapsel enthält:

| | |
|---|---|
| Kapselhülle: | Hartgelatinekapseln Größe 3 |

### Kapselinhaltsstoffe:

| | |
|---|---|
| (1) Wirksubstanz | 100,0 mg |
| (2) Lactose x 1H₂O | 38,0 mg |
| (3) Maisstärke getrocknet | 60,0 mg |
| (4) Magnesiumstearat | 2.0 mg |
| Kapselfüllgewicht: | 2̅0̅0̅,̅0̅ m̅g̅ |
| (5) Dest. Wasser | q.s. |

### Herstellung:

Ein kleiner Teil Lactose wird ca. 10 %ig in dest. Wasser gelöst (Granulierflüssigkeit). Der Wirkstoff, die restliche Lactose sowie Maisstärke werden gemischt und mit der Granulierflüssigkeit durchfeuchtet. Die Masse wird gesiebt, getrocknet und nach nochmaligem Sieben mit Magnesiumstearat homogen gemischt. Das feinkörnige Granulat wird auf einer geeigneten Maschine in Kapseln abgefüllt.

### Beispiel 9

### Hartgelatinekapseln zu 200 mg 4'-[2-[N-(2-(3-Chlor-phenyl)-2-hydroxy-ethyl)amino]propyl]-diphenylmethan-2-carbonsäureethylester

### Zusammensetzung:

| | |
|---|---|
| Kapselhülle: | Hartgelatinekapseln Größe 1 |

### Kapselinhaltsstoffe:

| | |
|---|---|
| (1) Wirksubstanz | 200,0 mg |
| (2) Lactose x 1H₂O | 47,0 mg |
| (3) Maisstärke getrocknet | 70,0 mg |
| (4) Magnesiumstearat | 3.0 mg |
| Kapselfüllgewicht: | 3̅2̅0̅,̅0̅ m̅g̅ |
| (5) Dest. Wasser | q.s. |

### Herstellung:

Ein kleiner Teil Lactose wird ca. 10 %ig in dest. Wasser gelöst (Granulierflüssigkeit). Der Wirkstoff, die restliche Lactose sowie Maisstärke werden gemischt und mit der Granulierflüssigkeit durchfeuchtet. Die Masse wird gesiebt, getrocknet und nach nochmaligem Sieben mit Magnesiumstearat homogen gemischt. Das feinkörnige Granulat wird auf einer geeigneten Maschine in Kapseln abgefüllt.

### Beispiel 10

### Alleinfutter II für Mastschweine

| | |
|---|---|
| Gerste | 370 g/kg |
| Weizennachmehl | 200 g/kg |
| Maniokmehl | 135 g/kg |
| Ackerbohnen | 100 g/kg |
| Raps-Extraktionsschrot | 100 g/kg |
| Futterfett | 65 g/kg |
| lysinreiches Mineralfutter für Schweine | 20 g/kg |
| Wirkstoff-Vormischung | 10 g/kg |

Diese Komponenten in den angegebenen Mengen ergeben nach sorgfältigem Mischen 1 kg Futter.

Die 10 g Wirkstoffvormischung enthalten z.B. 10 mg Wirkstoff und 9,99 g Maisstärke.

### Beispiel 11

### Mastfutter II für Broiler

| | |
|---|---|
| Mais | 625 g/kg |
| Sojabohnenmehl | 260 g/kg |
| Fleischmehl | 40 g/kg |
| Futterfett | 25 g/kg |
| Sojaöl | 17 g/kg |
| Bicalciumphosphat | 12 g/kg |
| Calciumcarbonat | 6 g/kg |
| Vitamin-/Mineralstoffmischung | 5 g/kg |
| Wirkstoff-Vormischung | 10 g/kg |

Diese Komponenten in den angegebenen Mengen ergeben nach sorgfältigem Mischen 1 kg Futter.

Die 10 g Wirkstoffvormischung enthalten z.B. 10 mg Wirkstoff und 9,99 g Maisstärke.

### Beispiel 12

### Kraftfutter für Rinder

| | |
|---|---|
| Zuckerrübenschnitzel | 600,0 g/kg |
| Maiskleber | 100,0 g/kg |
| Malzkeime | 50,0 g/kg |
| Sojabohnenmehl | 35,0 g/kg |
| Weizen | 110,0 g/kg |
| Melasse | 60,0 g/kg |
| Futterphosphate | 12,0 g/kg |
| Calciumcarbonat | 2,5 g/kg |
| Salz | 5,0 g/kg |
| Mineralstoffe | 10,0 g/kg |
| Vitamin-Vormischung | 5,5 g/kg |
| Wirkstoff-Vormischung | 10,0 g/kg |

Diese Komponenten in den angegebenen Mengen ergeben nach sorgfältigem Mischen 1 kg Futter.

Die 10 g Wirkstoffvormischung enthalten z.B. 10 mg Wirkstoff und 9,99 g Maisstärke.

### Beispiel 13

### Mastfutter für Lämmer

| | |
|---|---|
| Gerste | 690 g/kg |
| Sojabohnenmehl | 100 g/kg |
| Mais | 150 g/kg |
| Melasse | 30 g/kg |
| Vitamin-/Mineralstoffmischung | 20 g/kg |
| Wirkstoffvormischung | 10 g/kg |

Diese Komponenten in den angegebenen Mengen ergeben nach sorgfältigem Mischen 1 kg Futter.

Die 10 g Wirkstoffvormischung enthalten z.B. 10 mg Wirkstoff und 9,99 g Maisstärke.

## Patentansprüche

1. N-[2-(3-Chlor-phenyl)-2-hydroxy-ethyl]-N-(1-methyl-2-phenyl-ethyl)-amine der allgemeinen Formel 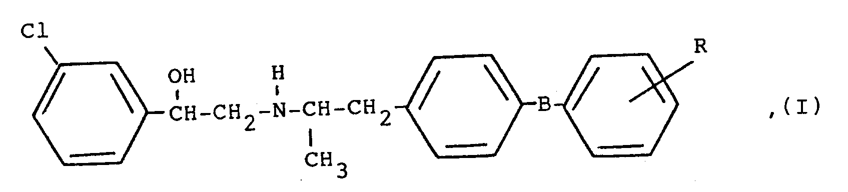 in der
B eine Bindung oder eine Methylengruppe und
R eine Methoxycarbonyl-, Ethoxycarbonyl-, 2-Hydroxy-ethoxy-, Methoxycarbonylmethoxy- oder Ethoxycarbonylmethoxygruppe in 2- oder 4-Stellung oder auch, wenn B eine Bindung darstellt und R in 4-Stellung steht, eine Carboxy- oder Carboxymethoxygruppe bedeuten, deren optische Isomere und deren Diastereomere sowie deren Additionssalze.

2. Folgende Verbindung gemäß Anspruch 1:
4'-[2-[N-(2-(3-Chlor-phenyl)-2-hydroxy-ethyl)amino]propyl]-4-(2-hydroxy-ethoxy)biphenyl, dessen optische Isomere und dessen Diastereomere sowie dessen Säureadditionssalze.

3. Folgende Verbindung gemäß Anspruch 1:
4'-[2-[N-(2-(3-Chlor-phenyl)-2-hydroxy-ethyl)amino]propyl]-biphenylyl-4-oxyessigsäure-methylester, dessen optische Isomere und dessen Diastereomere sowie dessen Säureadditionssalze.

4. Folgende Verbindung gemäß Anspruch 1:
4'-[2-[N-(2-(3-Chlor-phenyl)-2-hydroxy-ethyl)amino]propyl]-diphenylmethan-2-carbonsäure-ethylester, dessen optische Isomere und dessen Diastereomere sowie dessen Säureadditionssalze.

5. Physiologisch verträgliche Salze der Verbindungen gemäß den Ansprüchen 1 bis 4.

6. Arzneimittel, enthaltend eine Verbindung der Formel I nach mindestens einem der Ansprüche 1 bis 4 oder ein physiologisch verträgliches Additionssalz gemäß Anspruch 5 neben gegebenenfalls einem oder mehreren inerten Trägerstoffen und/oder Verdünnungsmitteln.

7. Verwendung einer Verbindung der Formel I nach mindestens einem der Ansprüche 1 bis 5 zur Herstellung eines Arzneimittels für die Behandlung des Diabetes mellitus, der Adipositas und zur Behandlung und Prophylaxe atherosklerotischer Veränderungen.

8. Verfahren zur Herstellung eines Arzneimittels gemäß Anspruch 6, dadurch gekennzeichnet, daß auf nichtchemischem Wege eine Verbindung der Formel I nach mindestens einem der Ansprüche 1 bis 5 in einen oder mehrere inerte Trägerstoffe und/oder Verdünnungsmittel eingearbeitet wird.

9. Leistungsförderndes Mittel für Tiere, enthaltend eine Verbindung der Formel I nach mindestens einem der Ansprüche 1 bis 5.

10. Verwendung einer Verbindung der Formel I nach mindestens einem der Ansprüche 1 bis 5 als leistungsförderndes Mittel.

11. Tiernahrung und Prämixe zur Herstellung von Tiernahrung, enthaltend eine Verbindung der Formel I nach mindestens einem der Ansprüche 1 bis 5.

12. Verfahren zur Herstellung von leistungsfördernden Mitteln für Tiere, dadurch gekennzeichnet, daß eine Verbindung der Formel I nach mindestens einem der Ansprüche 1 bis 5 mit Streck-, Verdünnungs- oder Nahrungsmitteln sowie gegebenenfalls mit weiteren Hilfsstoffen vermischt wird.

13. Verfahren zur Herstellung der neuen Phenylethanolamine gemäß den Ansprüchen 1 bis 5, dadurch gekennzeichnet, daß
a) eine Verbindung der allgemeinen Formel 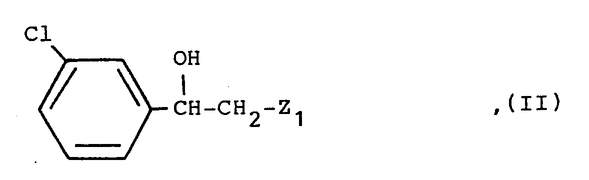 mit einer Verbindung der allgemeinen Formel 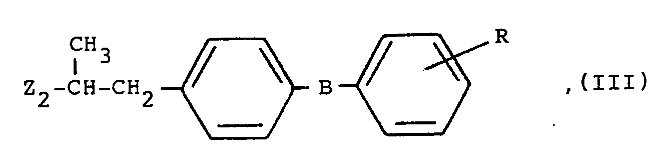 in denen
R und B wie in den Ansprüchen 1 bis 4 definiert sind, einer der Reste Z₁ oder Z₂ eine nukleophile Austrittsgruppe und
der andere der Reste Z₁ oder Z₂ eine Aminogruppe bedeuten, umgesetzt wird oder
b) eine gegebenenfalls im Reaktionsgemisch gebildete Schiff'-sche Base der allgemeinen Formel 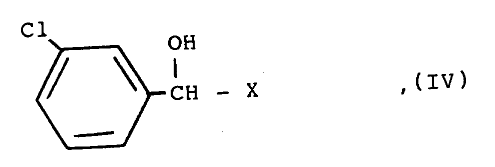 in der
X eine Gruppe der Formel 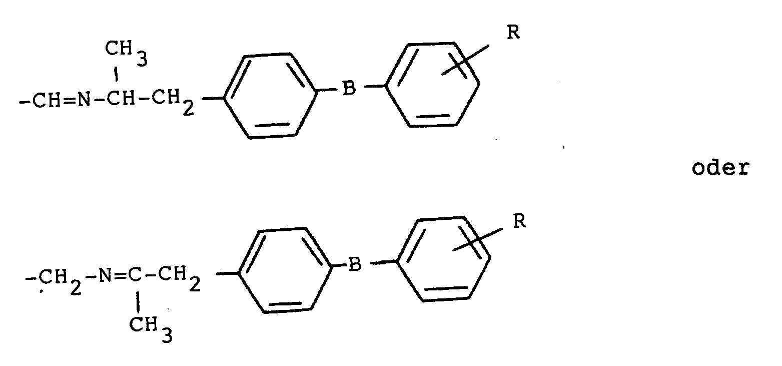 darstellt, wobei
R und B wie in den Ansprüchen 1 bis 4 definiert sind, reduziert wird oder
c) eine gegebenenfalls im Reaktionsgemisch gebildete Verbindung der allgemeinen Formel 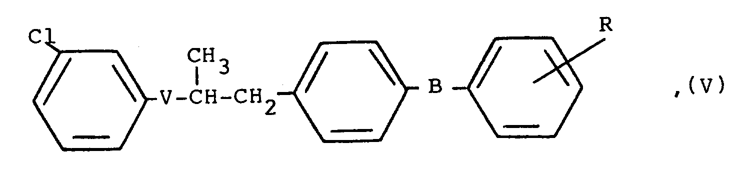 in der
R und B wie in den Ansprüchen 1 bis 4 definiert sind und
V eine Gruppe der Formel 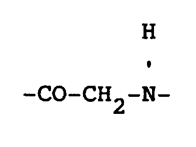 oder -CO-CH=N- darstellt, reduziert wird oder
d) eine Verbindung der allgemeinen Formel 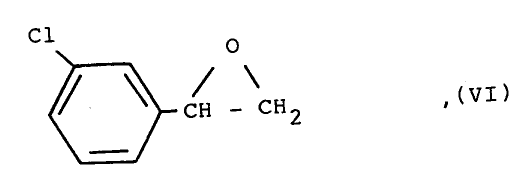 mit einem Amin der allgemeinen Formel 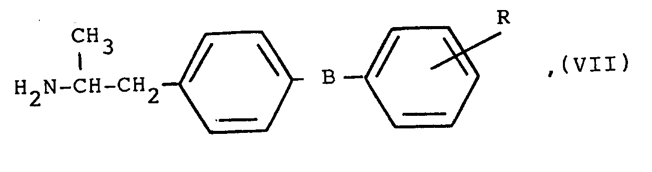 in der
R und B wie in den Ansprüchen 1 bis 4 definiert sind, umgesetzt wird oder
e) eine gegebenenfalls im Reaktionsgemisch gebildete Verbindung der allgemeinen Formel 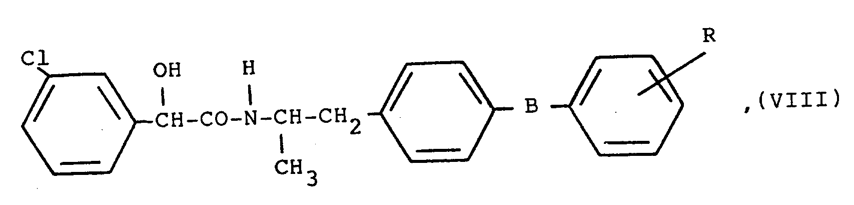 in der
R und B wie in den Ansprüchen 1 bis 4 definiert sind, reduziert wird oder
f) zur Herstellung von Verbindungen der allgemeinen Formel I, in der R eine 2-Hydroxy-ethoxygruppe darstellt, eine Verbindung der allgemeinen Formel 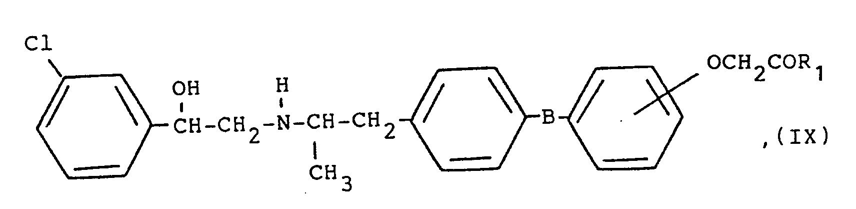 in der
B wie in den Ansprüchen 1 bis 4 definiert ist und
R₁ eine Hydroxy- oder Alkoxygruppe darstellt, reduziert wird oder
g) zur Herstellung von Verbindungen der allgemeinen Formel I, in der R eine Methoxycarbonyl-, Ethoxycarbonyl-, Methoxycarbonylmethoxy- oder Ethoxycarbonylmethoxygruppe in 2- oder 4-Stellung darstellt, eine Verbindung der allgemeinen Formel 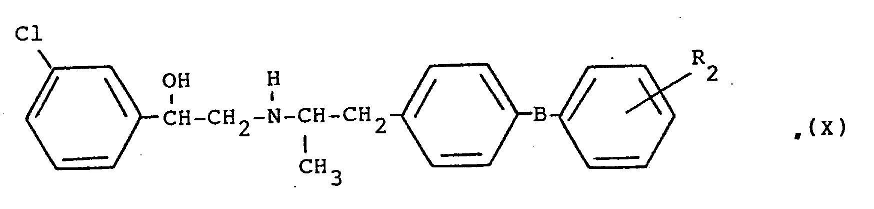 in der
B wie in den Ansprüchen 1 bis 4 definiert ist und
R₂ eine Carboxygruppe oder eine Carboxymethoxygruppe darstellt, oder deren reaktionsfähige Derivate mit einer Verbindung der allgemeinen Formel
H - R₃ ,(XI)
in der
R₃ eine Methoxy- oder Ethoxygruppe darstellt, umgesetzt wird und
erforderlichenfalls anschließend ein bei den Verfahren a) bis g) verwendeter Schutzrest abgespalten wird und/oder
gewünschtenfalls anschließend eine so erfindungsgemäß erhaltene Verbindung der Formel I, in der R eine Methoxycarbonyl- oder Ethoxycarbonylgruppe darstellt oder enthält, mittels Hydrolyse in eine entsprechende Verbindung der allgemeinen Formel I, in der R eine Carboxygruppe darstellt oder enthält, übergeführt wird und/oder
eine so erhaltene Verbindung der allgemeinen Formel I in ihre optischen Isomeren und Diastereomeren aufgetrennt wird und/oder
eine so erhaltene Verbindung der allgemeinen Formel I in ihre Additionssalze, insbesondere in ihre physiologisch verträglichen Additionssalze übergeführt wird.

## Claims

1. N-[2-(3-Chlorophenyl)-2hydroxyethyl]-N-(1-methyl-2-phenyl-ethyl)amines of general formula 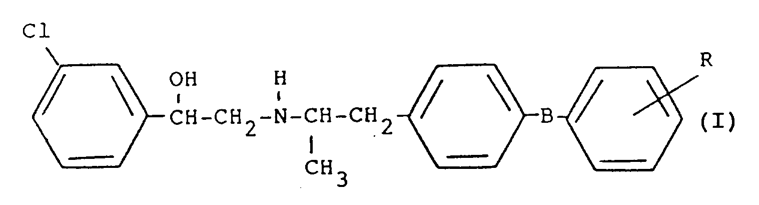 wherein
B represents a bond or a methylene group and
R denotes a methoxycarbonyl, ethoxycarbonyl, 2-hydroxyethoxy, methoxycarbonylmethoxy or ethoxycarbonylmethoxy group in the 2- or 4-position, or if B denotes a bond and R is in the 4-position, a carboxy or carboxymethoxy group,
the optical isomers and diastereoisomers and addition salts thereof.

2. The following compound according to claim 1:
4'-[2-[N-(2-(3-chloro-phenyl)-2-hydroxy-ethyl)amino]-propyl]-4-(2-hydroxy-ethoxy)biphenyl, the optical isomers and diastereoisomers and acid addition salts thereof.

3. The following compound according to claim 1:
methyl 4'-[2-[N-(2-(3-chloro-phenyl)-2-hydroxy-ethyl)-amino]propyl]biphenylyl-4-oxyacetate, the optical isomers and diastereoisomers and acid addition salts thereof.

4. The following compound according to claim 1:
ethyl 4'-[2-[N-(2-(3-chlorophenyl)-2hydroxy-ethyl)-amino]propyl]diphenylmethane-2-carboxylate, the optical isomers and diastereoisomers and acid addition salts thereof.

5. Physiologically acceptable salts of the compounds as claimed in claims 1 to 4.

6. A pharmaceutical composition containing a compound of formula I as claimed in at least one of claims 1 to 4 or a physiologically acceptable addition salt as claimed in claim 5 optionally together with one or more inert carriers and/or diluents.

7. Use of a compound of formula I as claimed in at least one of claims 1 to 5 for the manufacture of a pharmaceutical composition for the treatment of diabetes mellitus and obesity and for the treatment and prevention of atherosclerotic changes.

8. Process for preparing a pharmaceutical composition as claimed in claim 6, characterised in that, by a non-chemical method, a compound of formula I as claimed in at least one of claims 1 to 5 is incorporated in one or more inert carriers and/or diluents.

9. Performance enhancing substance for animals containing a compound of formula I as claimed in at least one of claims 1 to 5.

10. Use of a compound of formula I as claimed in at least one of claims 1 to 5 as a performance enhancing agent.

11. Animal feed and premixes for the preparation of animal feed, containing a compound of formula I as claimed in at least one of claims 1 to 5.

12. Process for preparing a performance enhancing substance for animals, characterised in that a compound of formula I as claimed in at least one of claims 1 to 5 is mixed with extenders, diluents or nutrients and optionally with other excipients.

13. Process for preparing the new phenylethanolamines as claimed in claims 1 to 5, characterised in that
a) a compound of general formula 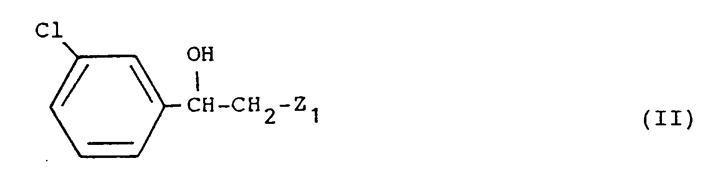 is reacted with a compound of general formula 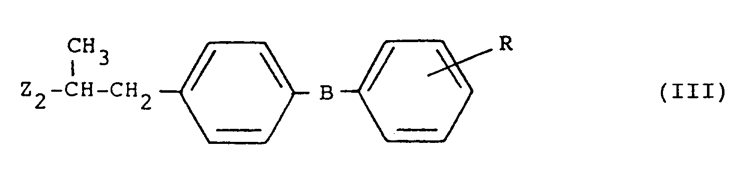 wherein
R and B are as defined in claims 1 to 4, one of the groups Z₁ and Z₂ is a nucleophilic leaving group and the other group Z₁ or Z₂ is an amino group or,
b) a Schiff base of general formula 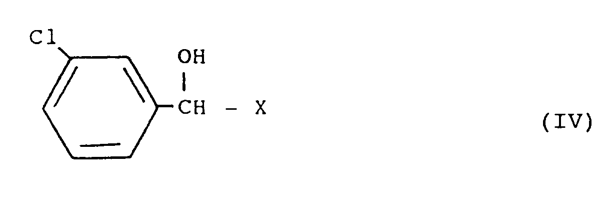 optionally formed in the reaction mixture
wherein
X represents a group of formula 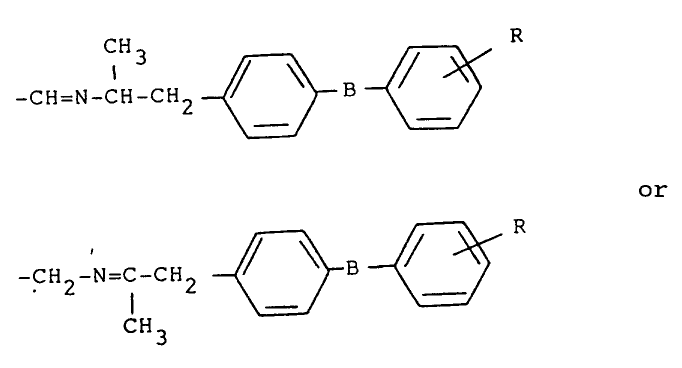 wherein
R and B are as defined in claims 1 to 4, is reduced, or
c) a compound of general formula 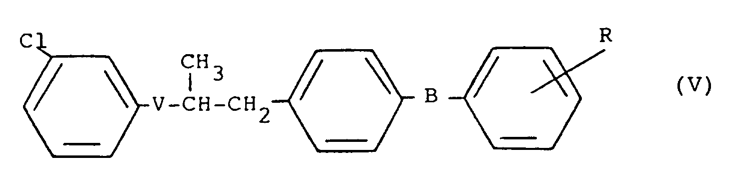 optionally formed in the reaction mixture,
wherein
R and B are as defined in claims 1 to 4 and
V represents a group of the formula 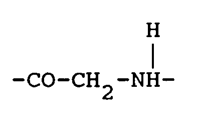 or
-CO-CH=N-, is reduced or
d) a compound of general formula 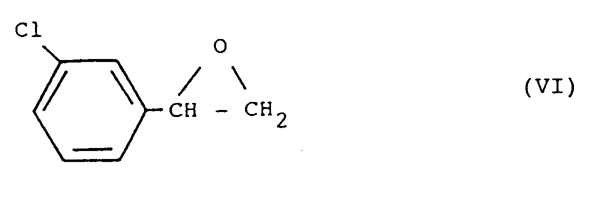 is reacted with an amine of general formula 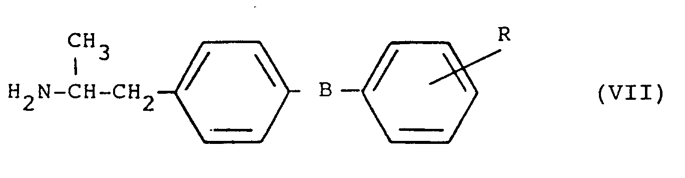 wherein
R and B are as defined in claims 1 to 4, or
e) a compound of general formula 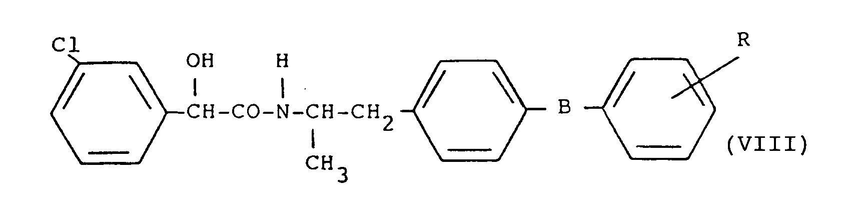 optionally formed in the reaction mixture, wherein R and B are as defined in claims 1 to 4, is reduced, or
f) in order to prepare compounds of general formula I wherein R denotes a 2-hydroxy-ethoxy group,
a compound of general formula 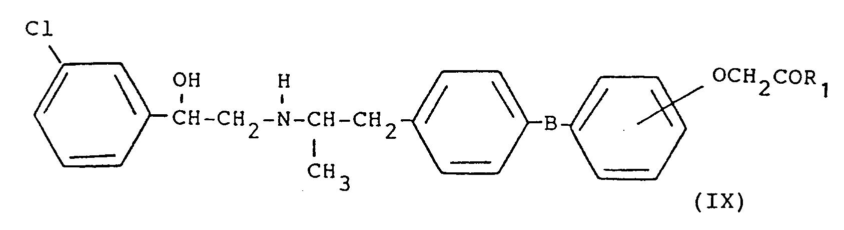 wherein
B is defined in claims 1 to 4 and
R₁ denotes a hydroxy or alkoxy group, is reduced, or
g) in order to prepare compounds of general formula I wherein R represents a methoxycarbonyl, ethoxycarbonyl, methoxycarbonylmethoxy or ethoxycarbonylmethoxy group in the 2- or 4-position,
a compound of general formula 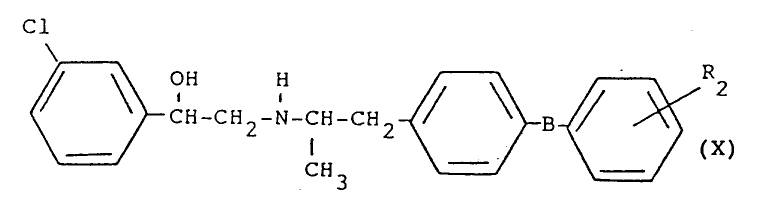 wherein
B is as defined in claims 1 to 4 and
R₂ represents a carboxy group or a carboxymethoxy group, or a reactive derivative thereof, is reacted with a compound of general formula
H - R₃ (XI)
wherein
R₃ represents a methoxy or ethoxy group, and
subsequently, if necessary, any protecting group used in process a) to g) is split off and/or
subsequently, if desired, a compound of formula I obtained according to the invention wherein R represents or contains a methoxycarbonyl or ethoxycarbonyl group, is converted by hydrolysis into a corresponding compound of general formula I wherein R represents or contains a carboxy group and/or
a compound of general formula I thus obtained is separated into its optical isomers and diastereoisomers and/or
a compound of general formula I thus obtained is converted into the addition salts thereof, particularly the physiologically acceptable addition salts thereof.

## Revendications

1. N-[2-(3-chlorophényl)-2-hydroxyéthyl]-N-(1-méthyl-2-phényléthyl)-amines de formule générale 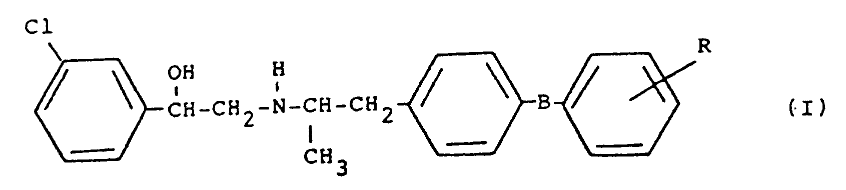 dans laquelle
B représente une liaison ou un groupe méthylène et
R représente un groupe méthoxycarbonyle, éthoxycarbonyle, 2-hydroxyéthoxy, méthoxycarbonylméthoxy ou éthoxycarbonylméthoxy en position 2 ou 4 ou bien, lorsque B représente une liaison et R est en position 4, un groupe carboxy ou carboxyméthoxy, leurs isomères optiques et leurs diastéréoisomères et leurs sels d'addition.

2. Composé suivant selon la revendication 1:
4'-[2-(N-(2-(3-chlorophényl)-2-hydroxyéthyl)amino]propyl]-4-(2-hydroxyéthoxy)biphényle, ses isomères optiques et ses diastéréoisomères, et ses sels d'addition d'acide.

3. Composé suivant selon la revendication 1:
4'-[2-[N-(2-(3-chlorophényl)-2-hydroxyéthyl)amino]propyl]-biphénylyl-4-oxyacétate de méthyle, ses isomères optiques et ses diastéréoisomères ainsi que ses sels d'addition d'acide.

4. Composé suivant selon la revendication 1:
4'-[2-[N-(2-(3-chlorophényl)-2-hydroxyéthyl)amino]propyl]-diphénylméthane-2-carboxylate d'éthyle, ses isomères optiques et ses diastéréoisomères ainsi que ses sels d'addition d'acide.

5. Sels physiologiquement acceptables des composés selon les revendications 1 à 4.

6. Médicament contenant un composé de formule I selon l'une au moins des revendications 1 à 4 ou un sel d'addition physiologiquement acceptable selon la revendication 5 et éventuellement un ou plusieurs véhicules et/ou diluants inertes.

7. Utilisation d'un composé de formule I selon l'une au moins des revendications 1 à 5 pour la préparation d'un médicament pour le traitement du diabète sucré, de l'adiposité et pour le traitement et la prophylaxie des modifications athérosclérotiques.

8. Procédé de préparation d'un médicament selon la revendication 6, caractérisé en ce qu'un composé de formule I selon l'une au moins des revendications 1 à 5 est incorporé par voie non chimique dans un ou plusieurs véhicules et/ou diluants inertes.

9. Agent favorisant les performances pour les animaux, contenant un composé de formule I selon l'une au moins des revendications 1 à 5.

10. Utilisation d'un composé de formule I selon l'une au moins des revendications 1 à 5 comme agent favorisant les performances.

11. Aliment pour animaux et prémélange pour la préparation d'aliments pour animaux, contenant un composé de formule I selon l'une au moins des revendications 1 à 5.

12. Procédé de préparation d'agents favorisant les performances pour les animaux, caractérisé en ce qu'un composé de formule I selon l'une au moins des revendications 1 à 5 est mélangé avec des délayants, des diluants ou des aliments et éventuellement avec d'autres adjuvants.

13. Procédé de préparation des nouvelles phényléthanolamines selon les revendications 1 à 5, caractérisé en ce que
a) un composé de formule générale 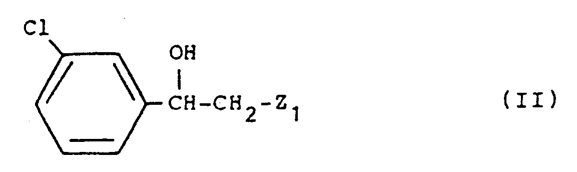 est mis à réagir avec un composé de formule générale 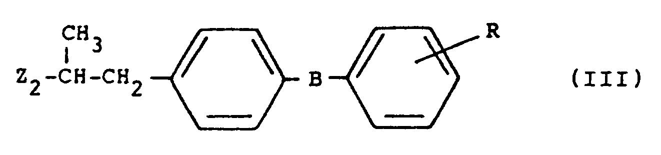 dans lesquelles
R et B sont définis comme dans les revendications 1 à 4, l'un des restes Z₁ et Z₂ représente un groupe partant nucléophile et
l'autre des restes Z₁ et Z₂ représente un groupe aminé, ou bien
b) une base de Schiff, éventuellement formée dans le mélange réactionnel, de formule générale 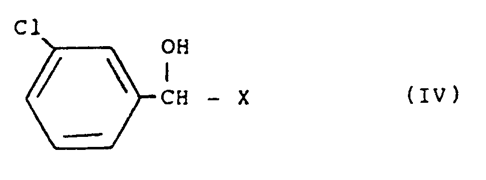 dans laquelle
X représente un groupe de formule 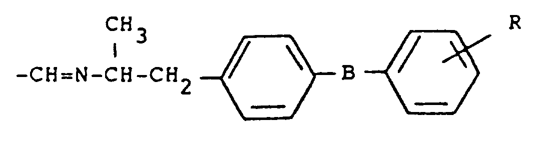 ou 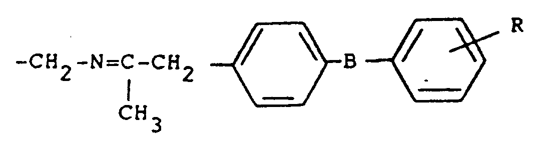 où R et B sont définis comme dans les revendications 1 à 4, est réduite, ou bien
c) un composé, éventuellement formé dans le mélange réactionnel, de formule générale 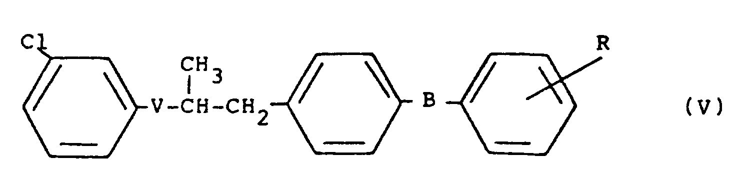 dans laquelle
R et B sont définis comme dans les revendications 1 à 4 et V représente un groupe de formule 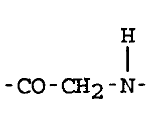 ou -CO-CH=N-,
est réduit, ou bien
d) un composé de formule générale 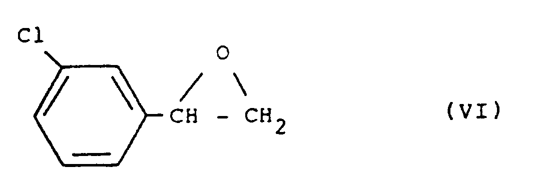 est mis à réagir avec une amine de formule générale 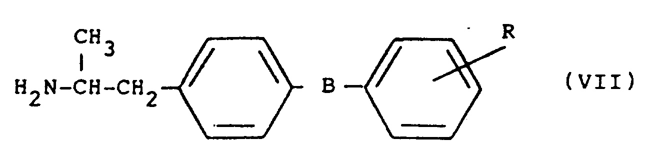 dans laquelle
R et B sont définis comme dans les revendications 1 à 4, ou bien
e) un composé, éventuellement formé dans le mélange réactionnel, de formule générale 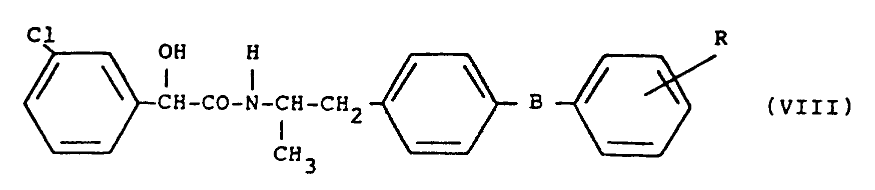 dans laquelle
R et B sont définis comme dans les revendications 1 à 4, est réduit, ou bien
f) pour la préparation de composés de formule générale I dans laquelle R représente un groupe 2-hydroryéthoxy, un composé de formule générale 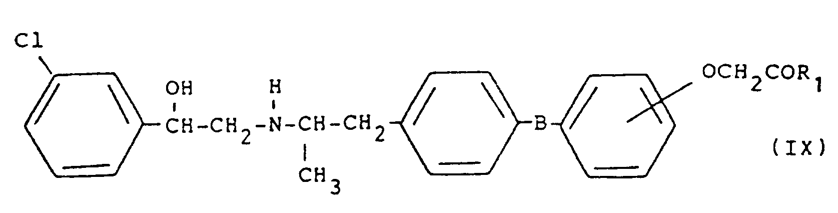 dans laquelle
B est défini comme dans les revendications 1 à 4 et R₁ représente un groupe hydroryle ou alcoxy, est réduit, ou bien
g) pour la préparation de composés de formule générale I dans laquelle R représente un groupe méthoxycarbonyle, éthoxycarbonyle, méthoxycarbonylméthoxy ou éthoxycarbonylméthoxy en position 2 ou 4, un composé de formule générale 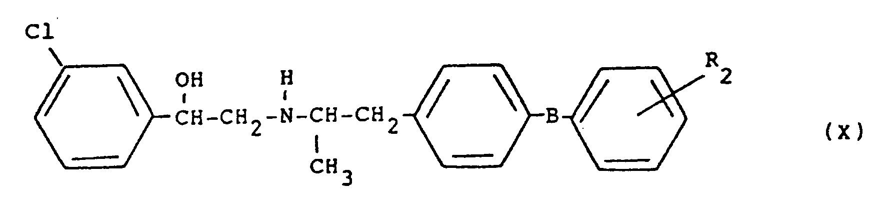 dans laquelle
B est défini comme dans les revendications 1 à 4 et R₂ représente un groupe carboxyle ou un groupe carboxyméthoxy, ou ses dérivés capables de réagir est mis à réagir avec un composé de formule générale
H - R₃ (XI)
dans laquelle
R₃ représente un groupe méthoxy ou éthoxy, et
si nécessaire un reste protecteur utilisé dans les procédés a) à g) est ensuite clivé et/ou si on le souhaite un composé ainsi obtenu selon l'invention, de formule I dans laquelle R représente ou contient un groupe méthoxycarbonyle ou éthoxycarbonyle est converti par hydrolyse en un compose correspondant de formule générale I dans laquelle R représente ou contient un groupe carboxy, et/ou
un composé de formule générale I ainsi obtenu est dédoublé en ses isomères optiques et diastéréoisomères et/ou
un composé de formule générale I ainsi obtenu est converti en ses sels d'addition, en particulier en ses sels d'addition physiologiquement acceptables.
